(19) Europäisches Patentamt
European Patent Office
Office européen des brevets

(11) Publication number: 0 248 227
A1

(12) **EUROPEAN PATENT APPLICATION**

(21) Application number: 87106614.8

(22) Date of filing: 07.05.87

(51) Int. Cl.[4]: C12N 15/00 , C12N 9/70 , C12P 21/02 , A61K 37/54

(30) Priority: 08.05.86 US 860960

(43) Date of publication of application: 09.12.87 Bulletin 87/50

(84) Designated Contracting States: AT BE CH DE ES FR GB GR IT LI LU NL SE

(71) Applicant: PHILLIPS PETROLEUM COMPANY
5th and Keeler
Bartlesville Oklahoma 74004(US)

(72) Inventor: Hagenson, Mary Jane
2208 SE Kristin Ln.
Bartlesville Oklahoma 74006(US)
Inventor: Stroman, David Womack
2504 SW Centre Rd.
Bartlesville Oklahoma 74003(US)

(74) Representative: Dost, Wolfgang, Dr. rer. nat. Dipl.-Chem. et al
Patent- u. Rechtsanwälte Bardehle, Pagenberg, Dost, Altenburg Frohwitter & Partner Galileiplatz 1
D-8000 München 80(DE)

(54) **Yeast production of streptokinase.**

(57) Novel DNA constructs comprising regulatory regions plus the structural coding region for streptokinase, which structural coding region is free of bacterial signal sequences, are disclosed. These novel constructs are incorporated into a variety of linear and circular plasmids. Such plasmids are used for yeast transformation and ultimately for the production of streptokinase by yeast.

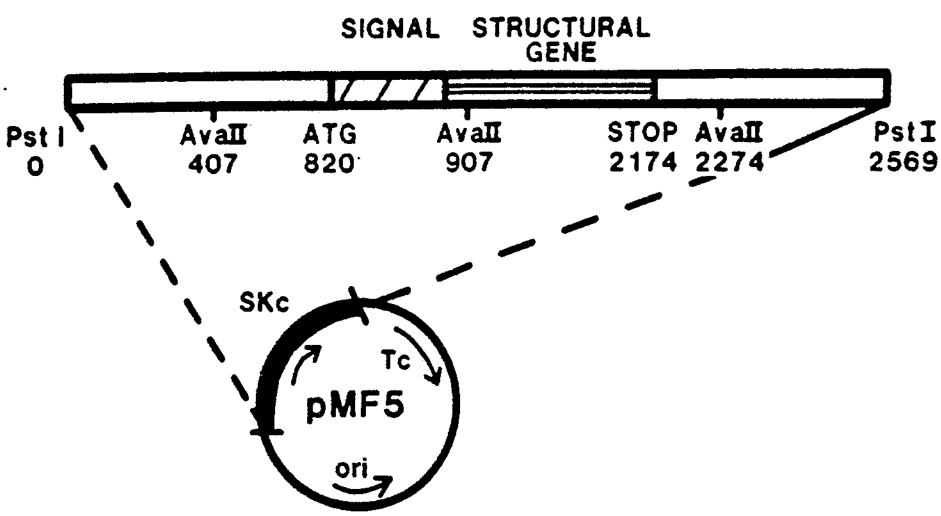


FIG. 6

EP 0 248 227 A1

Xerox Copy Centre

# YEAST PRODUCTION OF STREPTOKINASE

This invention relates to the use of recombinant DNA technology for the production of streptokinase. In one aspect, the present invention relates to the expression of streptokinase by yeast. In another aspect, the present invention relates to novel DNA constructs encoding streptokinase. In yet another aspect, the present invention relates to novel organisms transformed with the above described DNA constructs.

## BACKGROUND

As recombinant DNA technology has developed in recent years, the controlled production by microorganisms of an enormous variety of useful polypeptides has become possible. Many eukaryotic polypeptides, such as for example, human growth hormone, leukocyte interferons, human insulin and human proinsulin have already been produced by microorganisms. The continued application of techniques already in hand is expected in the future to permit production by microorganisms of a variety of other useful polypeptide products. One such useful polypeptide product is streptokinase.

Streptokinases are a well defined group of proteins exported by many strains of hemolytic streptococci to the growth medium. It was established some time ago that streptokinases play a role in the dissolution of blood clots and particularly of exudates which are rich in fibrin components. Depending on the nature and size of these clots, streptokinase in the presence of human plasminogen effects the dissolution of such clots. The dissolution of clots occurs because the streptokinases interact stoichiometrically with the enzymatically inert plasma plasminogen to yield the active enzyme plasmin. The plasmin so formed then degrades the fibrin network, by limited proteolysis, to form soluble products.

Prior to our application of recombinant DNA technology to the problem of large scale production of streptokinase, streptokinase was obtained as a fermentation product of hemolytic streptococci. Heretofore, the use of streptokinase has been limited by the fact that the streptokinase product isolated from streptococci which naturally produce streptokinase has been contaminated with extraneous streptococcal protein by-products which gave rise to toxic reactions in man. These reactions manifest themselves by increase in body temperature, fall in blood presure, shaking, chills, anoxia, cyanosis and the like. Thus, means have not been available for obtaining and maintaining sufficient supplies of streptokinase of sufficient purity for use in the treatment of blood clots. Hence, a method for the production of streptokinase in substantial quantities and free of streptococcal protein by-products at a reasonable cost would be of great value.

## OBJECTS OF THE INVENTION

An object of the invention, therefore, is a method for the production of streptokinase in yeast.

Another object of the present invention is the preparation of novel DNA constructs which are capable of expressing streptokinase in yeast at high levels.

These and other objects of the invention will become apparent from the disclosure and claims herein provided.

## STATEMENT OF THE INVENTION

In accordance with the present invention, we have discovered that streptokinase can be produced by yeast in high yields by culturing yeast cells transformed with DNA constructs comprising streptokinase coding regions under the control of yeast regulatory regions, wherein the streptokinase coding regions are free of bacterial signal sequences.

## BRIEF DESCRIPTION OF THE FIGURES

Figure 1 is a restriction map of the region 5′ of the primary dihydroxyacetone synthase gene from *Pichia* (DAS1).

Figure 2 is a restriction map of the region 5′ of the primary alcohol oxidase gene from *Pichia* (AOX1).

Figure 3 is a restriction map of the region 5′ of the p40 gene from *Pichia*.

Figure 4 is a restriction map of the autonomous replication sequence PARS1.

Figure 5 is a restriction map of the autonomous replication sequence PARS2.

Figure 6 is a restriction map of plasmid pMF5, including detail on the streptokinase coding region employed for modification and insertion into the *Pichia* expression vector pTHBS3V.

Figure 7 is a restriction map of the plasmid pTHBS3V.

Figure 8 is a restriction map of the plasmid pHTskc25.

The following abbreviations are used in the Figures for the restriction enzymes:

| Abbreviation | Restriction Enzyme |
|---|---|
| A | *Alu*I |
| Ah | *Aha*III |
| Av | *Ava*I |
| B | *Bam*HI |
| $B_2$ | *Bgl*II |
| Bc | *Bcl*II |
| C | *Cla*I |
| $H_2$ | *Hinc*II |
| $H_3$ | *Hind*III |
| K | *Kpn*I |
| Mb | *Mbo*II |
| $Nd_1$ | *Nde*I |
| Nr | *Nru*I |
| Ps | *Pst*I |
| $Pv_1$ | *Pvu*I |
| $R_1$ | *Eco*RI |
| $R_5$ | *Eco*RV |
| Rs | *Rsa*I |
| S | *Sal*I |
| Sm | *Sma*I |
| Ss | *Sst*I |
| T | *Taq*I |
| Xh | *Xho*I |

## DETAILED DESCRIPTION OF THE INVENTION

In accordance with the present invention, there is provided a novel DNA fragment comprising a yeast regulatory region and a polypeptide coding region wherein the polypeptide coding region codes for streptokinase or portions thereof, but where the coding region for streptokinase is free of bacterial signal sequences, and wherein the yeast regulatory region is capable of controlling the transcription of messenger RNA when positioned at the 5′-end of the streptokinase encoding region. Optionally, the novel DNA fragments of the present invention can also include a 3′ sequence of DNA downstream of the polypeptide

coding region, wherein the 3′ sequence of DNA is capable of controlling the polyadenylation and termination of transcription of messenger RNA coded for by the polypeptide coding region. The combination of regulatory region, streptokinase gene, and the transcriptional terminator fragment is referred to hereinafter as an expression cassette or expression unit.

Further in accordance with the present invention, there are provided novel linear and circular plasmids containing the above-described expression cassettes.

Still further in accordance with the present invention, there are provided essentially pure cultures of yeast strains transformed with the above-described linear or circular plasmids. In accordance with another embodiment of the present invention, process for preparing streptokinase is described which comprises cultivating a yeast strain transformed with the above-described plasmids under conditions where expression of the desired protein product is obtained.

Streptokinases are produced by various members of the *Streptococcus* family of bacteria. The streptokinase gene from *Streptococcus equisimilis* has been cloned and characterized by Malke and Ferretti (PNAS USA 81 , pages 3557-3561 (1984)) and Malke, Roe and Ferretti (Gene, 34 , pages 357-362 (1985)). The above cited references disclose the nucleotide sequence for the isolated streptokinase gene, including bacterial non-coding sequences positioned both 5′ and 3′ of the streptokinase coding region.

The streptokinase gene described by Malke et. al. was modified by cleavage with the restriction enzyme *Ava*II. This digestion removed the native 5′ signal sequences, the first ten nucleotides which code for the mature form of streptokinase, as well as some sequences 3′ of the streptokinase coding region. This truncated streptokinase coding sequence was then ligated with the synthetic *Ava* II-*Bam*HI linker shown below in Sequence A:

SEQUENCE A

```
     ↓          ↓     ↓                                  ↓
5'-GAT   CCC   GGG   AAT   TCC   ATG   ATT   GCT   G-3'
         GG    CCC   TTA   AGG   TAC   TAA   CGA   CCT  G
         ↑      ↑           ↑                            ↑
       BamHI    |         EcoRI                        AvaII
               SmaI
```

When the synthetic linker shown in Sequence A was ligated to the *Ava*II treated streptokinase coding fragment, the first ten nucleotides which had been deleted from the streptokinase coding region were replaced thereby providing four codons which encode the same amino acids as do the native sequences; and, in addition, an ATG initiation codon was provided. The DNA was subsequently digested with *Eco*RI to provide compatible sticky ends for ligation into the unique *Eco*RI site of the yeast expression vector pTHBS3V (see Figure 7). The resulting streptokinase coding sequence has the 5′-end nucleotide sequences and amino terminal translated amino acid sequences set forth below as Sequence B;

SEQUENCE B

| | | | | | | |
|---|---|---|---|---|---|---|
| Nucleotide Sequence | 5'-*ATG* | *ATT* | *GCT* | *GGA* | CCT....native streptokinase | |
| | 3'-*TAC* | *TAA* | *CGA* | *CCT* | GGA.... | sequences |
| Restriction endonuclease recognition sites | | | | | *Ava*II | |
| Translated sequence | Met | $Ile_1$ | $Ala_2$ | $Gly_3$ | $Pro_4$ . . . . | |

The nucleotide sequences (and translated amino acid sequences) obtained as a result of ligating Sequence A (shown below in italics) to the 3'-end of the truncated streptokinase coding fragment are set forth below in Sequence C:

SEQUENCE C

| | | | | |
|---|---|---|---|---|
| Nucleotide Sequence | 5'-GAC | AAA | TAA | CCACGGTCTT |
| | 3'-CTG | TTT | ATT | GGTGCCAGAA |
| | $Asp_{413}$ | $Lys_{414}$ | Stop | |

| | | | |
|---|---|---|---|
| CTAAAACGAT | GAGATTAACT | GACAAAAAAA | GCAAGCAACA |
| GATTTTGCTA | CTCTAATTGA | CTGTTTTTTT | CGTTCGTTGT |
| TGCTATCAAC | AGTTGCTTGC | TTTTTTCTAA | CCTCTTAGTT |
| ACGATAGTTG | TCAACGAACG | AAAAAAGATT | GGAGAATCAA |
| GTAGAGACTA | GTGACATTTC | GTGTCTAAAA | TAATCGTAAC |
| CATCTCTGAT | CACTGTAAAG | CACAGATTTT | ATTAGCATTG |
| TG*GTCCAGCA* | *ATCATGGAAT* | *TCCCGG* | |
| ACCAG*GTCGT* | *TAGTACCTTA* | *AGGGCCCGAT* | |

The complete streptokinase coding region employed for the expression work described in greater detail below has about 1245 nucleotides, a restriction map of which is presented in Figure 6 (Se the *Ava*II fragment which includes most of the streptokinase structural gene and some 3' sequences and runs from position 907 to 2274). This 1245 base pair fragment has been completely sequenced, and the sequence is set forth below as Sequence D;

SEQUENCE D

5'-AATTCC ATG ATT GCT GGA CCT GAG TGG CTG CTA GAC CGT CCA
TCT GTC AAC AAC AGC CAA TTA GTT GTT AGC GTT GCT GGT ACT
GTT GAG GGG ACG AAT CAA GAC ATT AGT CTT AAA TTT TTT GAA
ATC GAT CTA ACA TCA CGA CCT GCT CAT GGA GGA AAG ACA GAG
CAA GGC TTA AGT CCA AAA TCA AAA CCA TTT GCT ACT GAT AGT
GGC GCG ATG TCA CAT AAA CTT GAG AAA GCT GAC TTA CTA AAG
GCT ATT CAA GAA CAA TTG ATC GCT AAC GTC CAC AGT AAC GAC
GAC TAC TTT GAG GTC ATT GAT TTT GCA AGC GAT GCA ACC ATT
ACT GAT CGA AAC GGC AAG GTC TAC TTT GCT GAC AAA GAT GGT
TCG GTA ACC TTG CCG ACC CAA CCT GTC CAA GAA TTT TTG CTA
AGC GGA CAT GTG CGC GTT AGA CCA TAT AAA GAA AAA CCA ATA
CAA AAC CAA GCG AAA TCT GTT GAT GTG GAA TAT ACT GTA CAG
TTT ACT CCC TTA AAC CCT GAT GAC GAT TTC AGA CCA GGT CTC
AAA GAT ACT AAG CTA TTG AAA ACA CTA GCT ATC GGT GAC ACC
ATC ACA TCT CAA GAA TTA CTA GCT CAA GCA CAA AGC ATT TTA
AAC AAA AAC CAC CCA GGC TAT ACG ATT TAT GAA CGT GAC TCC
TCA ATC GTC ACT CAT GAC AAT GAC ATT TTC CGT ACG ATT TTA
CCA ATG GAT CAA GAG TTT ACT TAC CGT GTT AAA AAT CGG GAA
CAA GCT TAT AGG ATC AAT AAA AAA TCT GGT CTG AAT GAA GAA
ATA AAC AAC ACT GAC CTG ATC TCT GAG AAA TAT TAC GTC CTT
AAA AAA GGG GAA AAG CCG TAT GAT CCC TTT GAT CGC AGT CAC
TTG AAA CTG TTC ACC ATC AAA TAC GTT GAT GTC GAT ACC AAC
GAA TTG CTA AAA AGT GAG CAG CTC TTA ACA GCT AGC GAA CGT
AAC TTA GAC TTC AGA GAT TTA TAC GAT CCT CGT GAT AAG GTC
AAA CTA CTC TAC AAC AAT CTC GAT GCT TTT GGT ATT ATG GAC
TAT ACC TTA ACT GGA AAA GTA GAG GAT AAT CAC GAT GAC ACC
AAC CGT ATC ATA ACC GTT TAT ATG GGC AAG CGA CCC GAA GGA
GAG AAT GTC AGC TAT CAT TTA GCC TAT GAT AAA GAT CGT TAT
ACC GAA GAA GAA CGA GAA GTT TAC AGC TAC CTG CGT TAT ACA
GGG ACA CCT ATA CCT GAT AAC CCT AAC GAC AAA TAA CCACGGT
CTTCTAAAAC GATGAGATTA ACTGACAAAA AAAGCAAGCA ACATGCTATC
AACAGTTGCT TGCTTTTTTC TAACCTCTTA GTTGTAGAGA CTAGTGACAT
TTCGTGTCTA AAATAATCGT AACTGGTCCA GCAATCATGG-3'

Any yeast regulatory region is suitable for use in the practice of the present invention. The term "regulatory region" as used in this disclosure is intended to include the various functions necessary for controlled gene expression, e.g., promoter regions, upstream activator sequences, catabolite sensitive regions, and the like. Those of skill in the art are aware of numerous regulatory regions which have been characterized and which could be employed in conjunction with the modified streptokinase coding region prepared as described above.

Exemplary yeast regulatory regions include the acid phosphatase, alpha-mating factor and glyceraldehyde 3-phosphate dehydrogenase regulatory regions isolated from *Saccharomyces cerevisiae*; the primary alcohol oxidase (AOX1) and dihydroxyacetone synthase (DAS1) and p40 regulatory regions isolated from *Pichia pastoris*; the *Pichia* HIS4 regulatory region; and the like.

Presently preferred regulatory regions employed in the practice of the present invention are characterized by their ability to respond to media-containing:

(1) methanol,

(2) non-catabolite repressing carbon sources such as, for example, glycerol, galactose, acetate, and the like,

(3) catabolite repressing carbon sources, such as, for example, glucose, ethanol, fructose, and the like, followed by carbon source starvation.

Examples of these presently preferred regulatory regions are depicted by the restriction maps set forth in Figures 1, 2 and 3. The regulatory region depicted in Figure 1 is derived from the primary dihydroxyacetone synthase (DAS1) gene of *Pichia pastoris*. The regulatory region depicted in Figure 2 is derived from the primary alcohol oxidase (AOX1) gene of *Pichia pastoris* (*Pichia* has two alcohol oxidase genes, referred to hereinafter as AOX1 and AOX2). The regulatory region depicted in Figure 3 is derived from the p40 gene of *Pichia pastoris* . Those of skill in the art recognize that other regulatory regions having the above-described properties can be isolated from methylotrophic yeasts, such as for example, *Pichia pastoris*. Such additional regulatory regions having regulatory properties similar to the properties of the above-described regulatory regions are also within contemplation of the present invention.

The ligation of any of the above described regulatory regions with the truncated streptokinase gene is readily accomplished by those of skill in the art by application of known techniques. Note that the combination of regulatory region-linker (e.g., see Sequence A)-truncated coding sequence provides to the resulting construct the necessary ATG start codon required for protein expression, as well as the ten nucleotides which were removed when the "full" streptokinase gene was truncated, as described above.

The regulatory region-structural gene constructs of the present invention can be supplied to organisms for amplification, reproduction and expression in a variety of ways. In accordance with the present invention, it has been found that the initial ligation product can be transformed directly into a yeast host for amplicication and selection. Thus, instead of transforming the initial ligation mixture first into an *E. coli* host for amplification and selection, we prefer to transform directly into a yeast host.

Yeast transformants containing the desired streptokinase encoding constructs can be selected by applying appropriate selection pressure to the mixture of yeast cultures which results upon initial yeast transformation. Plasmid DNA can then be recovered from the selected yeast transformants and used to transform *E. coli*, on which appropriate assays are performed to verify the presence of streptokinase encoding sequences in the selected transformants. (Assays employed are described in Example X.)

For autonomous replication of the gene constructs in yeast, it is useful to employ an autonomous replication sequence (ARS) element as part of transforming DNA. Examples include PARS1 and PARS2 derived from *Pichia pastoris* as shown in Figures 4 and 5, respectively.

Where integrative transformation of the host is instead desired, no ARS element will be employed. A preferred method to achieve integrative transformation involves employing a site directed integration vector which comprises

a first insertable DNA fragment,

a streptokinase coding region free of bacterial signal sequences as described above,

a selectable marker gene, and

a second insertable DNA fragment.

The first and second insertable DNA fragments are each at least about 200 nucleotides in length and have nucleotide sequences which are homologous to portions of the genomic DNA of species of the genus *Pichia*. The various components of the integrative vector are serially arranged forming a linear fragment of DNA such that the expression cassette and the selectable marker gene are positioned between the 3′ end of the first insertable DNA fragment and the 5′ end of the second insertable DNA fragment. The first and second insertable DNA fragments are oriented with respect to one another in the serially arranged linear fragment as they are so oriented in the genome of *Pichia*.

It is necessary to include at least one selectable marker gene in the DNA use to transform the host strain. This facilitates selection and isolation of those organisms which the host did not have, e.g., restoration of the ability to produce a specific amino acid where the untransformed host strain has a defect in the specific amino acid biosynthetic pathway.

Those of skill in the art recognize that additional DNA sequences can also be incorporated into the vectors employed in the practice of the present invention, such as for example, bacterial plasmid DNA, bacteriophage DNA, and the like. Such sequences enable the amplification and maintenance of these vectors in bacterial hosts.

Expression in Transformed Yeast

The above-described plasmids of the present invention have utility in yeast strains which can be transformed. Regulation of streptokinase expression in yeast by the novel DNA constructs of the present invention can be accomplished by growth of the transformed strains under appropriate inducing or non-inducing conditions. For example, gene expression employing the presently preferred regulatory regions depicted in Figures 1, 2 and 3 can be accomplished by subjecting the transformed organisms to carbon source starvation. Carbon source starvation after growth on a variety of both catabolite repressing and non-catabolite repressing carbon sources induces expression of the gene product maintained under the control of the presently preferred regulatory regions of the invention. Another means to achieve expression of the desired gene product in appropriate species of transformed yeast is to grow transformed yeasts on methanol. Yet another means to induce expression of the desired gene product is to grow transformed yeast on media containing non-catabolite repressing carbon sources.

The presently preferred regulatory regions of this invention are useful for expression in all yeast strains, since these regulatory regions have been shown to be induced under a variety of conditions. Thus, yeasts capable of growth on methanol or on non-catabolite repressing carbon sources can be caused to produce streptokinase directly; while yeasts capable of growth on catabolite repressing carbon sources can be caused to produce streptokinase by subjecting yeast cells so grown to conditions of carbon source starvation.

Transformed yeast strains which are preferred for use in the process of the present invention employing various regulatory region-coding sequence constructs include members of the genera:

*Candida,*
*Kloeckera,*
*Saccharomyces,*
*Schizosaccharomyces,*
*Rhodotorula,*
*Hansenula,*
*Torulopsis,*
*Pichia, and*
*Kluyveromyces.*

Yeasts from these genera are preferred because their safety of handling, growth conditions and the like have been established and are well known to those of skill in the art.

Especially preferred yeast strains for use in the production of streptokinase in one embodiment of the present invention are those yeast strains which are capable of growth on methanol as carbon and energy source as such hosts are able to well utilize the presently preferred methanol responsive regulatory regions described above. Yeasts known to be capable of growth on methanol include members of the genera:

*Candida,*
*Kloeckera,*
*Saccharomyces,*
*Rhodotorula,*
*Hansenula,*
*Torulopsis, and*
*Pichia.*

Since the presently preferred regulatory regions of the present invention are also induced by growth on non-catabolite repressing carbon sources as well as conditions of carbon source starvation, yeast strains which are capable of growth on such non-methanolic substrates as:

glucose,
acetate,

glycerol,
ethanol,
lactose,
galactose,
fructose,
sucrose,

and the like and mixtures of any two or more thereof are also useful in the practice of the invention. By growing the host organism on a suitable non-catabolite repressible, non-methanolic carbon source such as, for example, glycerol or galactose, or by growing the host organism on a suitable catabolite repressible carbon source such as, for example, ethanol, glucose and fructose, then subjecting the host organism to carbon source starvation conditions, expression of streptokinase under the control of the presently preferred regulatory regions of the invention can be achieved.

An especially preferred host yeast strain is the mutant *Pichia pastoris* GS115, which is a mutant defective in the ability to produce histidine. GS115 has been designated as having the mutant genotype *his4* , as a result of the defect in the histidine pthway affecting the histidinol dehydrogenase-encoding gene. GS115 is derived from *Pichia pastoris* NRRL Y-11430 and has been deposited with the Northern Regional Research Center of the United States Department of Agriculture in Peoria, Illinois, and has been assigned the accession number NRRL Y-15851. This particular host is useful because it is an auxotrophic mutant deficient in the histidine pathway. Transformation of this host with a vector containing, among other DNA sequences, sequences encoding the HIS4 gene function, allows ready selection of transformed hosts.

Another preferred yeast strain for use in the practice of the present invention is the mutant *Pichia pastoris* GS190, which is a mutant defective in the arginine pathway affecting the argininosuccinate lyase encoding gene. GS190 is derived from *Pichia pastoris* NRRL Y-11430, and has been deposited with the Northern Regional Research Center of the United States Department of Agriculture in Peoria, Illinois, and has been assigned the accession number NRRL Y-18014.

Yet another preferred host yeast strain is the double auxotrophic mutant PPF1, which is a mutant defective in both the histidine and arginine pathways. PPF1 is defective in both the histidine pathway affecting the histidinol dehydrogenase encoding gene and the arginine pathway affecting the argininosuccinate lyase encoding gene. PPF1 has been deposited with the Northern Regional Research Center of the United States Department of Agriculture in Peoria, Illinois, and has been assigned the accession number NRRL Y-18017.

*Escherichia coli* is also a suitable host for the plasmids of the invention. Those of skill in the art recognize that many strains of *E. coli* are readily available and are suitable hosts.

## *Pichia pastoris* Transformation Procedure

The experimental procedures for the transformation of *Pichia pastoris* have been previously described, and are presented in greater detail below (Example I).

*Pichia Pastoris* can be transformed by enzymatic digestion of the cell walls to give spheroplasts; the spheroplasts are then mixed with the transforming DNA and incubated in the presence of calcium ions and polyethylene glycol, then regenerated in selective growth medium deficient in histidine. The transforming DNA includes the HIS4 gene in which the host strain is deficient, thus only transformed cells survive on the selective growth medium employed.

The desired product, streptokinase, is produced by growth of transformed cells under conditions under which the regulatory region induces expression of the polypeptide coding region encoding streptokinase. Those of skill in the art are capable of readily determining such appropriate cultivation conditions without the need for carrying out undue experimentation.

Once the growth of cells and expression of streptokinase has been carried to acceptable levels, product can be recovered employing a variety of protein recovery means known to those of skill in the art. Exemplary methods include cell disruption by such as vigorous mixing with glass beads, followed by centrifugation to yield a soluble protein extract.

The isolated protein-containing fraction can be assayed in a variety of ways, for example, by plasminogen activation assays, by polyacrylamide gel electrophoresis of the cellular proteins, and by immunoreaction with streptokinase antibody. These assays are described in greater detail in the Examples.

The invention will now be described in greater detail with reference to the following non-limiting examples.

EXAMPLES

General information pertinent throughout the Examples:

Strains

*Pichia pastoris* GTS115 (*his4* ) [pNRRL Y-15851] was the host yeast strain used in these Examples.

*E. coli* K-12 strains 848 (*F[-] met thi gal tonA hsdR hsdM*) and *E. coli* GM119 (*dam dcm*) were used for propagation of plasmid DNAs.

Plasmids

Plasmid pMF5 is illustrated in Figure 6. Malke and Ferretti (PNAS USA 81, pp. 3557-3561 (1984)) and Malke, Roe and Ferretti (Gene 34, pp. 357-362 (1985)).

Plasmid PTHBS3V is illustrated in Figure 7. Plasmid pTHBS3V contains *Pichia* HIS4, PARS1 and 5'-AOX1 sequences, which can be obtained from plasmid pSAOH5 (which is available in an *E. coli* host from the Northern Regional Research Center of the United States Department of Agriculture, Peoria, Illinois, with the accession number NRRL B-15862). The 3'AOX1 sequences can be obtained from plasmid pPG4.0 (which is available in an *E. coli* host from the USDA with accession number NRRL B-15868). Alternatively, pTHBS3V can be prepared from pHTskc25 (available from USDA in an *E. coli* host with accession number NRRL B-18069) by *Eco*RI digestion, then re-ligation of the opened vector minus the streptokinase sequences (which are present in pHTskc25 as an *Eco*RI insert).

Plasmid Isolations

Plasmids were isolated from *E. coli* using the procedure of Birnboim and Dolby (Nucl. Acids Res. 7, pp. 1513-1523 (1979)). Plasmid DNA from *Pichia pastoris* was isolated as described previously by Cregg (Mol. Cell. Biol. 5, 3376 (1985)).

Enzymes and Chemicals

Streptokinase purchased from Boehringer Mannheim Biochemicals was used as a standard. Human serum plasminogen was obtained from CalBiochem. Restriction endonucleases were from New England Biolabs. Acrylamide, bisacrylamide, TEMED, and ammonium persulfate were obtained from BioRad. T4 DNA ligase was from Promega, and polynucleotide kinase and calf intestinal phosphatase were purchased from Boehringer Mannheim Biochemicals. $^{125}$I-protein A was obtained from Amersham and agamma calf serum was from Gibco.

Antibody to Streptokinase

Rabbit antisera against purified *S. equisimilis* H46A streptokinase was kindly provided by Drs. J. J. Ferretti and M. L. Dau (University of Oklahoma).

Media

*E. coli* were grown in LB medium with 10 µg/mL tetracycline or 50 µg/mL ampicillin.

*Pichia pastoris* shake flask cultures were grown in IM3 media with YTM4 trace minerals. Carbon sources were 2.0% glucose, 1.0% glycerol, or 0.5% methanol.

The buffers and solutions employed in the following examples have the compositions given below:
1M Tris buffer 121.1 g Tris base in 800 mL of $H_2O$;
adjust pH to the desired value by adding concentrated (35%) aqueous HCl;
allow solution to cool to room temperature before final pH adjustment, dilute to a final volume of 1L.

TE buffer 1.0 m_M_ EDTA
in 0.01 _M_ (pH 7.4) Tris buffer
PBS (Phosphate buffered saline) 10 m_M_ sodium phosphate (pH 7.0)
0.15 _M_ NaC1
SDS Gel Loading Buffer 62.5 m_M_ Tris-HCl (pH 6.8)
2% SDS
10% glycerol
100 m_M_ dithiothreitol
0.001% bromphenol blue
YPD Medium 1% Bacto-yeast extract
2% Bacto-peptone
2% Dextrose
SD Medium 6.75 g yeast nitrogen base without amino acids (DIFCO)
2% Dextrose
in 1 L of water
SED 1 _M_ Sorbitol
25 m_M_ EDTA
50 m_M_ DTT
SCE Buffer 9.1 g Sorbitol
1.47 g Sodium citrate
0.168 g EDTA
50 mL $H_2O$
--pH to 5.8 with HCl
CaS 1 _M_ Sorbitol
10 m_M_ $CaCl_2$
--filter sterilize
PEG Solution 20% polyethylene glycol-3350
10m_M_ $CaCl_2$
10m_M_ Tris-HCl (pH 7.4)
--filter sterilize
SOS 1 _M_ Sorbitol
0.3x YPD medium
10 m_M_ $CaCl_2$
FM-21 Salts Medium
$H_3PO_4$(85%) 3.5 mL
$CaSO_4 \bullet 2H_2O$ 0.15 g
$K_2SO_4$ 2.38 g
$MgSO_4 \bullet 7H_2O$ 1.95 g
KOH 0.65 g
$FeSO_4 \bullet 7H_2O$ 0.065 g
$CuSO_4 \bullet 5H_2O$ 0.006 g
$ZnSO_4 \bullet 7H_2O$ 0.020 g
$MnSO_4 \bullet H_2O$ 0.003 g
Biotin 0.000041 g
1L water
IM3 Salts Medium
$KH_2PO_4$ 15.0 g
$K_2HPO_4$ 1.0 g
$MgSO_4 \bullet 7H_2O$ 0.50 g
$CaSO_4 \bullet 2H_2O$ 0.04 g
$(NH_4)_2SO_4$ 3.00 g
Biotin 0.00005 g
pH to 5.4 1 L water
YTM-4 Trace Minerals Concentrate
$FeSO_4 \bullet 7H_2O$ 65.0 g
$CuSO_4 \bullet 5H_2O$ 6.0 g

$ZnSO_4 \bullet 7H_2O$ 20.0 g
$MnSO_4 \bullet H_2O$ 3.0 g
1L water
Add 250 µL/liter of Media

EXAMPLE I

*Pichia pastoris* Transformation Procedure

A. Cell Growth

1. Inoculate a colony of *Pichia pastoris* GS115 (NRRL Y-15851) into about 10 mL of YPD medium and shake culture at 30°C for 12-20 hours.

2. After about 12-20 hours., dilute cells to an $OD_{600}$ of about 0.01-0.1 and maintain cells in log growth phase in YPD medium at 30°C for about 6-8 hours.

3. After about 6-8 hours, inoculate 100 mL of YPD medium with 0.5 mL of the seed culture at an $OD_{600}$ of about 0.1 (or equivalent amount). Shake at 30°C for about 12-20 hours.

4. Harvest culture when $OD_{600}$ is about 0.2-0.3 (after approximately 16-20 hours) by centrifugation at 1500 g for 5 minutes.

B. Preparation of Spheroplasts

1. Wash cells once in 10 mL of sterile water. (All centrifugations for steps 1-5 are at 1500 g for 5 minutes.)

2. Wash cells once in 10 mL of freshly prepared SED.

3. Wash cells once in 10 mL of sterile 1 M Sorbitol.

4. Resuspend cells in 10 mL SCE buffer.

5. Add 10 µL of 3 mg/mL Zymolyase 60,000 (Miles Laboratories). Incubate cells at 30°C for about 10 minutes.

6. Wash spheroplasts once in 10 mL of sterile 1 M Sorbitol by centrifugation at 1000 g for 5-10 minutes. (The time and speed for centrifugation may vary; centrifuge enough to pellet spheroplasts but not so much that they rupture from the force.)

7. Wash cells once in 10 mL of sterile CaS.

8. Resuspend cells in total of 3.5 mL of CaS.

C. Transformation

1. Add DNA samples (up to 20 µL volume) to 12 X 75 mm sterile polypropylene tubes. (DNA should be in water or TE buffer; for maximum transformation frequencies with small amounts of DNA, it is advisable to add about 1 µL of 5 mg/mL sonicated *E. coli* DNA to each sample.)

2. Add 100 µL of spheroplasts to each DNA sample and incubate at room temperature for about 20 minutes.

3. Add 1 mL of PEG solution to each sample and incubate at room temperature for about 15 minutes.

D. Regeneration of Spheroplasts

1. Recipe for Regeneration Agar Medium:

a. Agar-KCl-9 g Bacto-agar, 13.4 g KCl, 240 mL $H_2O$ autoclave.

b. 10X Glucose-20 g Dextrose, 100 mL $H_2O$, autoclave.

c. 10X SC-6.75 g Yeast Nitrogen Base without amino acids, 100 mL $H_2O$, autoclave. (Add any desired amino acid or nucleic acid up to a concentration of 200 µg/mL before or after autoclaving.)

d. Add 30 mL of 10X Glucose and 20 mL of 10X SC to 300 mL of the melted Agar-KCl solution. Add 0.6 mL of 0.2 mg/mL biotin and any other desired amino acid or nucleic acid to a concentration of 20 µg/mL. Hold melted Regeneration Agar at 55-60°C.

2. Plating of Transformation Samples:

Pour bottom agar layer of 10 mL Regeneration Agar per plate at least 30 minutes before transformation samples are ready. Distribute 10 mL aliquots of Regeneration Agar to tubes in a 45-50°C bath during the period that transformation samples are in SOS. Add 50, 250 or 700 µL aliquots of the transformed sample to 10 mL aliquots of melted Regeneration Agar held at 45-50°C and pour each onto plates containing a solid 10 mL bottom agar layer of Regeneration Agar.

3. Incubate plates at 30°C for 3-5 days.

## EXAMPLE II

### Transformation of *E. coli*

The method of Hanahan (J. Mol. Biol. 166, p. 557 (1983)) was used to transform all strains of *E. coli*.

## EXAMPLE III

### Synthetic Linker

A synthetic linker was designed for inserting a streptokinase structural gene, minus its signal peptide sequence, into a variety of expression vectors. The linker was synthesized as two complementary strands, the sequences for which are shown in Sequence A. The lyophylized oligomers were suspended in sterile 10 mM Tris, 1 mM EDTA. Equal molar volumes were mixed and heated to 90°C followed by slow cooling to room temperature to anneal the complementary strands. The double stranded linker has a *Bam*HI site at its 5′ end and an *Ava*II site at its 3′ end.

## EXAMPLE IV

### Inserting the Streptokinase Gene into Expression Vector pTHBS3V

The steps used in inserting the streptokinase structural gene, minus the signal peptide coding region, into the *Eco*RI site of expression vector pTHBS3V are described in this Example. Plasmid pMF5 (see Figure 6) was propagated in *E. coli* GM119 so that the purified plasmid DNA could be digested with *Ava*II, which is methylation sensitive. The 1370 bp *Ava*II fragment from pMF5 was recovered by preparative gel electrophoresis and electroelution in dialysis tubing. This *Ava*II fragment contains the coding sequence for all but the first four amino acids of the mature streptokinase protein and the signal peptide (26 amino acids). An ATG initiation codon and the coding sequence for the deleted first four amino acids of the mature protein were replaced by the synthetic linker. The *Ava* II fragment was ligated with excess linker DNA, extracted with phenol/chloroform, and ethanol precipitated. The ligated DNA was resuspended in restriction buffer and digested with *Eco*RI for subsequent inserting into the *Eco*RI site pTHBS3V. The vector had been previously cleaved at its unique *Eco*RI site for subsequent ligation with the *Eco*RI cleaved *Ava*II-linker (Sequence A) fragment. The vector after being digested with *Eco*RI was treated with calf intestinal phosphatase (CIP) to reduce the frequency of self-ligation. The ligated mixture of DNA was used to transform *Pichia pastoris* strain GTS115 directly.

## EXAMPLE V

Identification and Characterization of the Streptokinase Producing Clone

*Pichia pastoris* GTS115 cells transformed with the streptokinase-pTHBS3V ligation mixture prepared as described in Example IV were screened for histidine prototrophy. Fifty $His^+$ transformants were selected at random for plasmid isolation. One of the clones contained a plasmid of the expected size (9.6 kb)(Figure 8), while another clone which contained self-ligated pTHBS3V plasmid was carried along as a control in subsequent experiments. This clone, *Pichia pastoris* GTS115 (pHTskc25), has been deposited with the Northern Regional Research Center of the United States Department of Agriculture in Peoria, Illinois, in order to ensure access by the public upon issuance of this application as a patent. This strain has been assigned the accession number NRRL Y-18070. In order to confirm the nature of these two clones, plasmid DNA from the above-described clones was used to transform *E. coli* 848.

Since heterologous genes under control of the AOX1 promoter are expressed to some extent in *E. coli*, we screened the *E. coli* 848 transformants for streptokinase production. The *E. coli* 848 (pHTskc25) transformants showed a clearing zone in the casein overlay assay (see Example X), indicating the production of streptokinase by these cells, while *E. coli* transformants containing the vector pTHBS3V did not show zones of clearing in the casein overlay. *E. coli* bearing the plasmid pHTskc25 has been deposited with the Northern Regional Research Center of the United States Department of Agriculture in Peoria, Illinois, in order to ensure access by the public upon issuance of this application as a patent. This strain has the laboratory designation 848 (pHTskc25) and has been assigned accession number NRRL B-18069.

Plasmid DNA from *E. coli* 848 (pHTskc25) was examined by restriction endonuclease digestions with *Eco*RI. *Pst*I, *Sal*I, and *Hind*III. Restricted DNA fragment sizes confrmed that pHTskc25 is the desired streptokinase containing construct, with the *Ava*II fragment including the synthetic linkers in the proper orientation with respect to the AOX1 promoter.

EXAMPLE VI

The expression of streptokinase by GTS115 (pHTskc25) was studied in 20 mL shake flasks under growth conditions where the streptokinase gene was both repressed and derepressed. GTS115 (pTHBS3V) was included as a control in these studies. When grown on glycerol or glucose, the AOX1 promoter is repressed, while under carbon limitation or growth on methanol, the alcohol oxidase promoter is active. The growth rates for both strains were essentially the same for each carbon source, indicating that the presence and expression of the streptokinase gene had no detectable harmful or inhibitory effects on cell growth. The amount of streptokinase produced was determined by the BAEE assay (see Example X). There was no streptokinase produced during growth on glycerol or glucose, as expected. The methanol grown culture produced significant levels of streptokinase which reached 134 U/mL at an O.D. of 8.9. Production per cell appeared to be constant throughout cell growth at a level of about 16 U/O.D. cells.

EXAMPLE VII

Continuous Culture Studies

Production of streptokinase was examined in two continuous culture runs. Each run was performed using a 5-L New Brunswick Scientific fermentor equipped with monitors and controls for pH, dissolved oxygen (D.O.), agitator speed, temperature, air flow, and oxygen flow.

Each continuous culture was maintained at pH 5.0 by the addition of $NH_3$ gas into the air stream. Temperature was held at 30°C. Fermentor value ranged from 2.4 L to 1.8 L. Cell yields were determined from washed cell dry weights.

Inocula for the fermentor runs were grown in 250 mL Erlenmeyer flasks containing 100 mL of IM-3 salts and 2% w/v glycerol as sole source of carbon and energy. The fermentor cultures were grown in the batch mode with 2% w/v glycerol-IM-3 media until the available glycerol was exhausted. Continuous cultures were established using 10% w/v glycerol-FM21 salts feed as the nutrient source until steady-state conditions were achieved. Once baseline control samples were taken, the nutrient feed was switched from glycerol to 15% v/v methanol-FM21 salts with no intervening starvation period. Each continuous culture, regardless of carbon source, was run as a chemostat where the growth rate of the population was limited by the availability of the carbon source.

The first fermentor run was carried out for 15.5 hours on limiting methanol as sole source of carbon and energy. Five samples (A through E) were withdrawn from the culture effluent during the fermentation for analysis.

The production of streptokinase when cell growth in the fermentor was maintained at 19.4 grams/liter dry weight of cells is shown in Table 1.

TABLE 1
Streptokinase Produced During Fermentor Run of GTS115 (pHTskc25)

| Sample | Time (hrs) Relative to MeOH Chemostat | O.D.600 | Assays* µg skc/mL | Assays* Units skc/mL |
|---|---|---|---|---|
| A | -1(Glycerol Chemostat) | 103 | 1.94 | 208 |
| B | 0 (MeOH Feed) | 107 | 1.30 | 139 |
| C | 9.5 | 114 | 3.86 | 413 |
| D | 14.0 (Dry Wt = 25 g/l) | 112 | 31.6 | 3,381 |
| E | 15.5 (End of Run) | 104 | 60.0 | 6,420 |

*Cells were broken for two minutes in breaking buffer (no Triton X-100)

BAEE assays were done on cells lysed by agitation for two minutes (no Triton X-100). At the termination of the fermentation, after 15.5 hours on methanol, 6,420 U/mL (60.0 µg/mL) of streptokinase was present in the culture lysate. This is equivalent to about 61 U/O.D. of cells. During the last 1.5 hours of fermentation, the amount of streptokinase per milliliter doubled, while the cell density remained constant. This indicates that the culture was not fully induced at this point and production levels had not reached steady state.

Plasmid stability was determined on the basis of maintenance of the $His^+$ phenotype. Cells from samples A through E were washed with sterile water, diluted, and plated on IM3-glucose plates, either with or without histidine. In addition, colonies which grew in the presence of histidine were replica plated onto $His^-$ plates. Greater than 98% of the colonies were histidine prototrophs maintaining the His4 gene and, presumably, the streptokinase gene as well.

The second fermentor run was run for 255 hours on limiting methanol as sole source of carbon and energy. Eleven samples (A through M) were taken periodically for analysis.

During the second fermentation of GTS115 (pHTskc25), the culture was brought to 46 grams/liter dry weight and maintained at steady state (constant dilution rate) for 10 days. For BAEE assays on samples A through M, cells were broken by two-minute agitation (no Triton X-100). Streptokinase production in µg/mL and U/mL equivalents over the period of fermentation is summarized in Table 2.

TABLE 2

| Sample | Time (hrs.) in Methanol Chemostat | Streptokinase µg/mL | Streptokinase U/mL |
|---|---|---|---|
| A | -2 | 2.4 | 260 |
| B | 0 | 3.4 | 370 |
| C | 14 | 85 | 9,100 |
| D | 18 | 110 | 11,800 |
| E | 22 | 122 | 13,100 |
| H | 86.5 | 125 | 13,400 |
| K | 158.5 | 122 | 13,100 |
| M | 254.5 | 142 | 15,200 |

Streptokinase yield increased slightly from about 12,000 U/mL (112 µg/mL) to about 15,000 U/mL (140 µg/mL) as a result of the extended fermentation time.

Studies were then performed to determine the effect of longer agitation time and inclusion of Triton X-100 in the lysis media on the streptokinase yield. Results are summarized in Table 3.

TABLE 3

| Agitation Time, min. | Final Triton X-100 Concentration, % | Streptokinase µg/mL | Streptokinase U/mL |
|---|---|---|---|
| 2 | 0 | 142 | 15,200 |
| 3 | 0 | 274 | 29,300 |
| 4 | 0 | 260 | 27,800 |
| 2 | 0.005 | 256 | 27,400 |
| 3 | 0.005 | 274 | 29,300 |
| 4 | 0.005 | 246 | 26,300 |
| 3 | 0.01 | 240 | 25,700 |
| 4 | 0.01 | 246 | 26,322 |
| 3 | 0.02 | 212 | 22,700 |
| 4 | 0.02 | 192 | 21,000 |

The yield nearly doubled to about 29,300 U/mL (274 µg/mL) when cells were broken in the presence of 0.005% Triton X-100 or agitation was increased to three minutes. Further increases in cell agitation time and Triton concentration actually decreased the streptokinase yield.

Plasmid stability was determined as previously described for Sample M. Greater than 98% of cells from Sample M were histidine prototrophs, indicating a high degree of stability over the lengthy fermentation.

## EXAMPLE VIII

### Lysis of *Pichia* Cells for Streptokinase Assays

*Pichia* cells were broken by agitation in the presence of glass beads using a Minibead-beater (Biospec Products, Bartlesville, Oklahoma). *Pichia* cells were washed in sterile water and resuspended in an equal volume of sterile 0.2 M Tris-HCl, 0.1% BSA. Tubes (1.5 mL) were half-filled with washed 0.5 mm diameter glass beads fnd 1.0 mL of cells were added. Tubes were agitated for two to four minutes, and in some instances, Triton X-100 (0.005, 0.01, or 0.02%) was added in the breaking buffer.

## EXAMPLE IX

### Characterization of the Streptokinase Protein Produced in *Pichia* Yeast

Proteins from GTS115 (pHTskc25) fermentor run samples A and M were separated by polyacrylamide gel electrophoresis. GTS115 (pTHBS3V) proteins and authentic streptokinase were included as controls. Results are summarized as follows. From unconcentrated Sample M lysate there was a new 47 kD protein band which was not observed in the lysate from the control *Pichia* culture, GTS115 (pTHBS3V), which protein is of the right size to be streptokinase.

Proteins separated by polyacrylamide gel electrophoresis were analyzed for reaction with streptokinase antibody by Western blot analysis. As observed by radioactive imaging, a single radioactive band corresponding to a 47 kD protein is apparent in the GTS115 (pHTskc25) protein lane. There is no reaction of antibody with GTS115 (pTHBS3V) proteins. *Pichia* streptokinase was recognized by antibody produced against *S. equisimilis* streptokinase, indicating that the proteins produced in these diverse host organisms are antigenically similar. In addition, there is no cross reaction to other *Pichia* proteins with the antibody and no evidence for degraded forms of streptokinase.

## EXAMPLE X

### A. Casein Overlay for Detection of Streptokinase

The caseinolytic activity of plasmin produced by the activation of plasminogen by streptokinase made it possible to test individual colonies of *E. coli* for streptokinase production (6). Cells grown on culture plates were overlayed with about 10 ml of 1.0% agarose in 50 mM Tris-HCl, pH 8.0, 150 mM NaCl, 0.02 g/mL powdered milk, and 0.05 U/ml human plasminogen. Plates were incubated at 37°C and clearing zones resulting from casein lysis became apparent after at least two hours of incubation for streptokinase producing colonies.

To show that some other protease was not digesting the casein, casein overlays of *E. coli* 848 (pHTskc25) were done without plasminogen in the overlay. An active protease would yield clearing zones in either case, while streptokinase can cause casein digestion only in the presence of plasminogen. We observed no degradation of casein in the absence of plasminogen, verifying that the observed clearing zones were a result of the action of streptokinase on plasminogen.

B. Benzoyl-L-Arginine Ethylester (BAEE) Assay for Streptokinase

An assay for detection of nanogram quantities of streptokinase was developed which involved incubating samples to be analyzed for streptokinase activity with a predetermined amount of human plasminogen and then incubating for 15 minutes at room temperature. During this incubation period, streptokinase associates with plasminogen activating it to the enzyme plasmin. A given amount of BAEE substrate was then added which is hydrolyzed by plasmin, resulting in a proportional increase in absorbance at 253 nm. A dual beam spectrophotometer with a recorder (Perkin-Elmer model 559A) was used to plot the increase in absorbance relative to the blank (sample minus streptokinase). The change in otpical density per minute versus nanograms of streptokinase (over a range of 0 to 260 nanograms) was plotted using standard streptokinase. The plotting was linear over this range. Unknowns were diluted as needed to give OD/minute values which fell within this linear range of the standard curve.

C. SDS-Polyacrylamide Gel Electrophoresis

*Pichia* cells were broken as described above. Cell lysates were kept on ice until preparation for loading. Separating gels (10% acrylamide) with stacking gels (6% acrylamide) were used for separation of proteins according to standard procedures. Gels were stained with Coomassie Brilliant Blue for visualization of protein bands.

D. Western Blots for the Detection and Quantitation of Streptokinase Protein

Protein extracts were electrophoresed as described previously and transferred to nitrocellulose using a transblot apparatus (BioRad Laboratories) according to the company's specifications. The Western Blot procedure was carried out as follows: The blot was soaked for two hours in blocking buffer (20% agamma calf serum in Buffer A, which consists of 0.15 M NaCl, 50 MM Tris, pH 7.6). Streptokinase antibody was diluted in 25 mL of incubation buffer (5% agamma calf serum in Buffer A), placed in a fresh sealable bag with the nitrocellulose blot, and incubated at 25°C for two hours with gentle shaking. The blot was then washed in Buffer A, twice in Buffer A with 0.05% Triton X-100, and once again in Buffer A, each for 10 minutes using 200 ml. The blot was placed in a fresh bag to which 20 ml of incubation buffer with 20 µCi of $^{125}$I-protein A was added. The sealed bag was rocked for 1.5 hours at room temperature with several turnings of the bag to insure uniform coverage, followed by washing as described previously. The blot was dried, wrapped in saran wrap, and exposed to Kodak XAR-5 film with two intensifying screens at -80°C for at least two hours. Limit of detection was about 10 nanograms streptokinase protein.

E. Specific Activity of Streptokinase Protein

The actual specific activity of streptokinase is a poorly defined value, in part due to the difficulty in standardizing an appropriate substrate for enzymatic activity. Several different methods are used to define the unit of activity. We have elected to use the highest specific activity value reported in the literature, which is approximately 107,000 U/mg protein (Mol. Gen. Genet. *196*, 360, 1984).

The examples have been provided merely to illustrate the practice of our invention and should not be read so as to limit the scope of our invention or the appended claims in any way. Reasonable variations and modifications, not departing from the essence and spirit of our invention, are contemplated to be within the scope of patent protection desired and sought.

## Claims

1. A DNA fragment comprising:

(a) a yeast regulatory region which is capable of controlling the transcription of messenger RNA in yeast when positioned at the 5' end of a polypeptide encoding region, and

(b) a polypeptide coding region, wherein said coding region codes for streptokinase or portions thereof, and wherein said region coding for streptokinase is free of bacterial signal sequences.

2. The DNA fragment of claim 1 characterized in that said yeast regulatory region is selected from
the Pichia AOX1 regulatory region,
the Pichia DAS1 regulatory region,
the Pichia glyceraldehyde-3-phosphate dehydrogenase regulatory region,
the Saccharomyces acid phosphatase regulatory region,
the Saccharomyces alpha-mating factor regulatory region,
the Saccharomyces glyceraldehyde-3-phosphate dehydrogenase regulatory region, and
the Pichia p40 regulatory region.

3. The DNA fragment of claim 1 characterized in that said region coding for streptokinase is about 1250 base pairs in length and is characterized as shown by the AvaII fragment containing nucleotides 907 to 2274 in the restriction map presented in Figure 6; in particular wherein said region coding for streptokinase has the nucleotide sequence:

5'-GAATTCC ATG ATT GCT GGA CCT GAG TGG CTG CTA GAC CGT CCA
TCT GTC AAC AAC AGC CAA TTA GTT GTT AGC GTT GCT GGT ACT
GTT GAG GGG ACG AAT CAA GAC ATT AGT CTT AAA TTT TTT GAA
ATC GAT CTA ACA TCA CGA CCT GCT CAT GGA GGA AAG ACA GAG
CAA GGC TTA AGT CCA AAA TCA AAA CCA TTT GCT ACT GAT AGT
GGC GCG ATG TCA CAT AAA CTT GAG AAA GCT GAC TTA CTA AAG
GTC ATT CAA GAA CAA TTG ATC GCT AAC GTC CAC AGT AAC GAC
GAC TAC TTT GAG GTC ATT GAT TTT GCA AGC GAT GCA ACC ATT
ATC GAT CGA AAC GGC AAG GTC TAC TTT GTC GAC AAA GAT GGT
TCG GTA ACC TTG CCG ACC CAA CCT GTC CAA GAA TTT TTG CTA
AGC GGA CAT GTG CGC GTT AGA CCA TAT AAA GAA AAA CCA ATA
CAA AAC CAA GCG AAA TCT GTT GAT GTG GAA TAT ACT GTA CAG
TTT ACT CCC TTA AAC CCT GAT GAC GAT TTC AGA CCA GGT CTC
AAA GAT ACT AAG CTA TTG AAA ACA CTA GCT ATC GGT GAC ACC
ATC ACA TCT CAA GAA TTA CTA GCT CAA GCA CAA AGC ATT TTA
AAC AAA AAC CAC CCA GGC TAT ACG ATT TAT GAA CGT GAC TCC
TCA ATC GTC ACT CAT GAC AAT GAC ATT TTC CGT ACG ATT TTA
CCA ATG GAT CAA GAG TTT ACT TAC CGT GTT AAA AAT CGG GGA
CAA GCT TAT AGG ATC AAT AAA AAA TCT GGT CTG AAT GAA GAA
ATA AAC AAC ACT GAC CTG ATC TCT GAG AAA TAT TAC GTC CTT
AAA AAA GGG GAA AAG CCG TAT GAT CCC TTT GAT CGC AGT CAC
TTG AAA CTG TTC ACC ATC AAA TAC GTT GAT GTC GAT ACC AAC
GAA TTG CTA AAA AGT GAG CAG CTC TTA ACA GCT AGC GAA CGT
AAC TTA GAC TTC AGA GAT TTA TAC GAT CCT CGT GAT AAG GTC
AAA CTA CTC TAC AAC AAT CTC GAT GTC TTT GGT ATT ATG GAC
TAT ACC TAT ATC GGA AAA GTA GAG GAT AAT CAC GAT GAC ACC
AAC CGT ATC ATA ACC GTT TAT ATG GGC AAG CGA CCC GAA GGA
GAG AAT GTC AGC TAT CAT TTA GCC TAT GAT AAA GAT CGT TAT
ACC GAA GAA GAA CGA GAA GTT TAC AGC TAC CTG CGT TAT ACA

GGG ACA CCT AAT CCT GAT AAC CCT AAC GAC AAA TAA CCACGGT
CTTCTAAAAC GATGAGATTA ACTGACAAAA AAAGCAAGCA ACATGCTATC
AACAGTTGCT TGCTTTTTTC TAACCTCTTA GTTGTAGAGA CTAGTGACAT
TTCGTGTCTA AAATAATCGT AACTGGTCCA GCAATCATGG AATTC-3' ;

in particular wherein said yeast chromosomal DNA comprises an autonomously replicating DNA sequence and a marker gene.

4. The DNA fragment of claim 1 further comprising a 3' sequence of DNA downstream of the polypeptide coding region, wherein said 3' sequence of DNA is capable of controlling the polyadenylation and termination of transcription of messenger RNA coded for by said polypeptide coding region.

5. The DNA fragment of any of claims 1 to 4 characterized in that said DNA fragment further comprises one or more additional DNA sequences derived from

bacterial plasmid DNA,
bacteriophage DNA,
yeast plasmid DNA, and
yeast chromosomal DNA;

in particular wherein said yeast chromosomal DNA comprises an autonomously replicating DNA sequence and a marker gene.

6. The DNA fragment of claim 1 further comprising serially arranged DNA which comprises:

a first insertable DNA fragment,
a selectable marker gene, and
a second insertable DNA fragment;

wherein said first and second insertable DNA fragments are each at least about 200 nucleotides in length and have nucleotide sequences which are homologous with portions of the genomic DNA of species of the genus Pichia; wherein said DNA fragment, said marker gene and said DNA fragment of claim 1 are positioned between the 3' end of said first insertable DNA fragment and the 5' end of said second insertable DNA fragment; and wherein said first and second insertable DNA fragments are oriented with respect to one another as they are so oriented in the genome of Pichia.

7. A plasmid comprising:

the DNA fragment of claim 1,
bacterial plasmid DNA,
a selectable yeast marker gene, and
a yeast autonomous replication sequence;

in particular wherein said plasmid is the plasmid pHTskc25.

8. An essentially pure culture of a strain of the genus Pichia transformed with the DNA fragment of any of the preceding claims or transformed with the plasmid pHTskc25.

9. A process for preparing streptokinase characterized by cultivating a yeast strain transformed with the plasmid of claim 7 under conditions under which the regulatory region induces expression of the polypeptide coding region encoding streptokinase and, optionally, isolating and purifying said streptokinase.

10. A method for preparing regulatory region-streptokinase coding region constructs useful for expression of streptokinase in yeast characterized by

(a) removing the signal peptides from the streptokinase coding region obtained from S. equisimilis to produce a truncated streptokinase coding sequence,

(b) ligating the truncated streptokinase coding sequence with a synthetic linker sequence which contains an ATG codon and which restores the complete streptokinase coding sequence, and,

(c) ligating the streptokinase coding region prepared in accordance with step (b) with a yeast regulatory region; in particular wherein said signal peptides are removed from the streptokinase coding region obtained from S. equisimilis by digesting with AvaII; in particular wherein the ATG start site for the streptokinase coding region is provided by a synthetic polylinker having the nucleotide sequence:

```
5'-GAT CCC GGG AAT TCC ATG ATT GCT G-3'
      GG CCC TTA AGG TAC TAA CGA CCT G

    BamHI      EcoRI                 AvaII
      SmaI
```

in particular wherein said yeast regulatory region is selected from the group identified in claim 2.

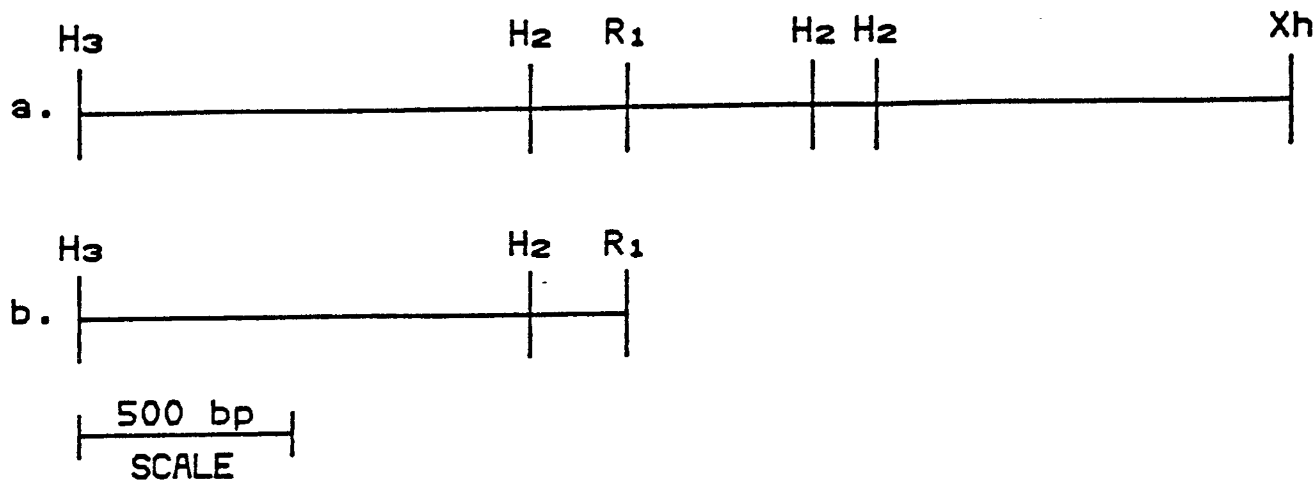


FIG. 1

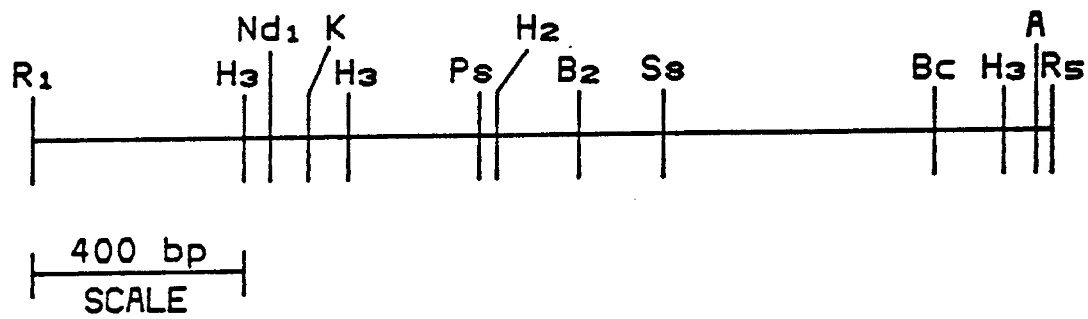


FIG. 2

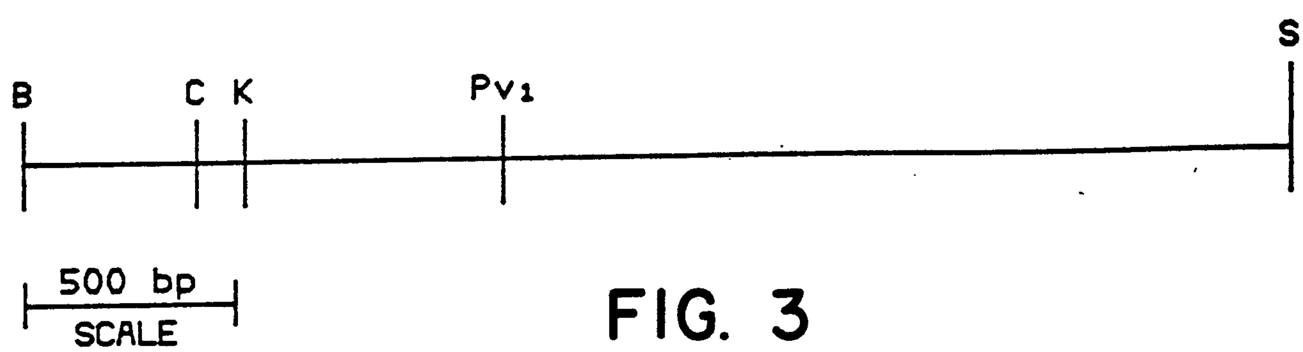


FIG. 3

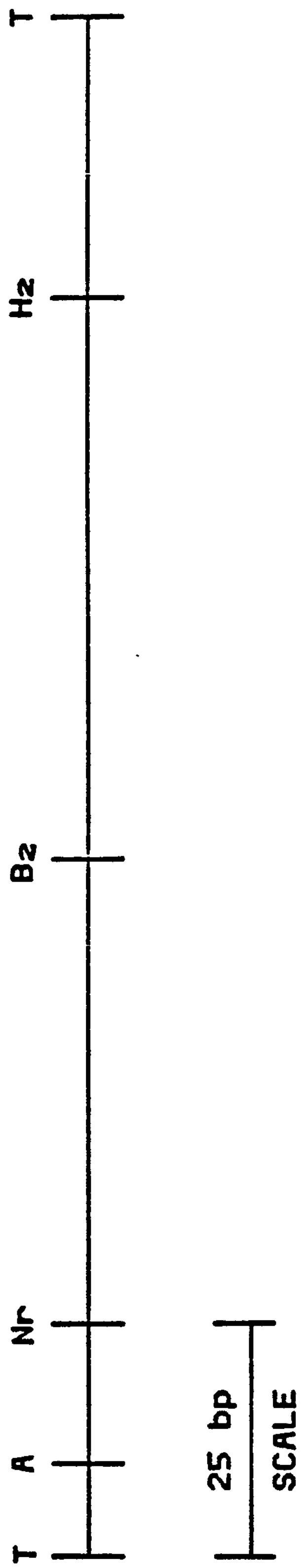


FIG. 4

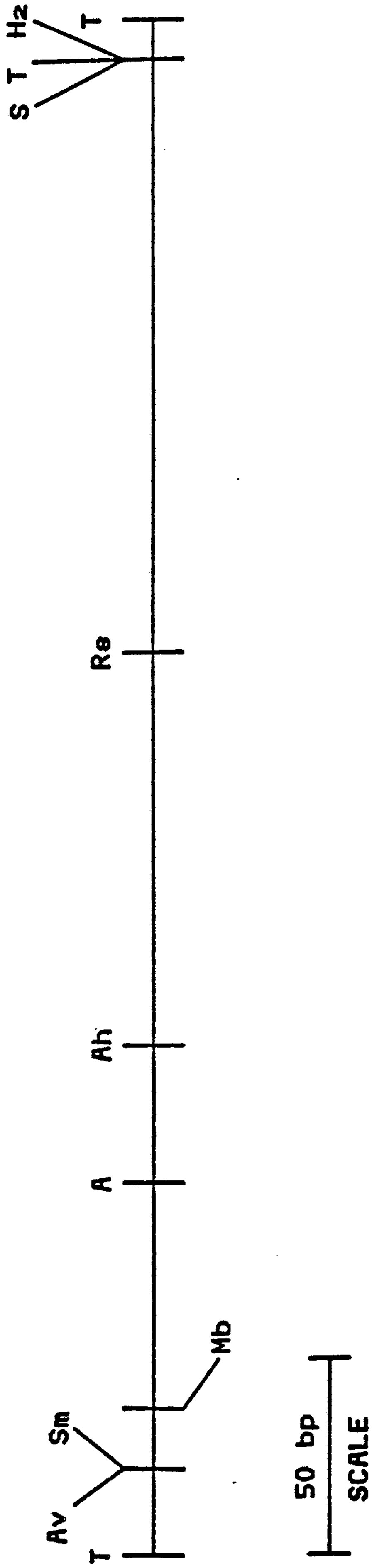


FIG. 5

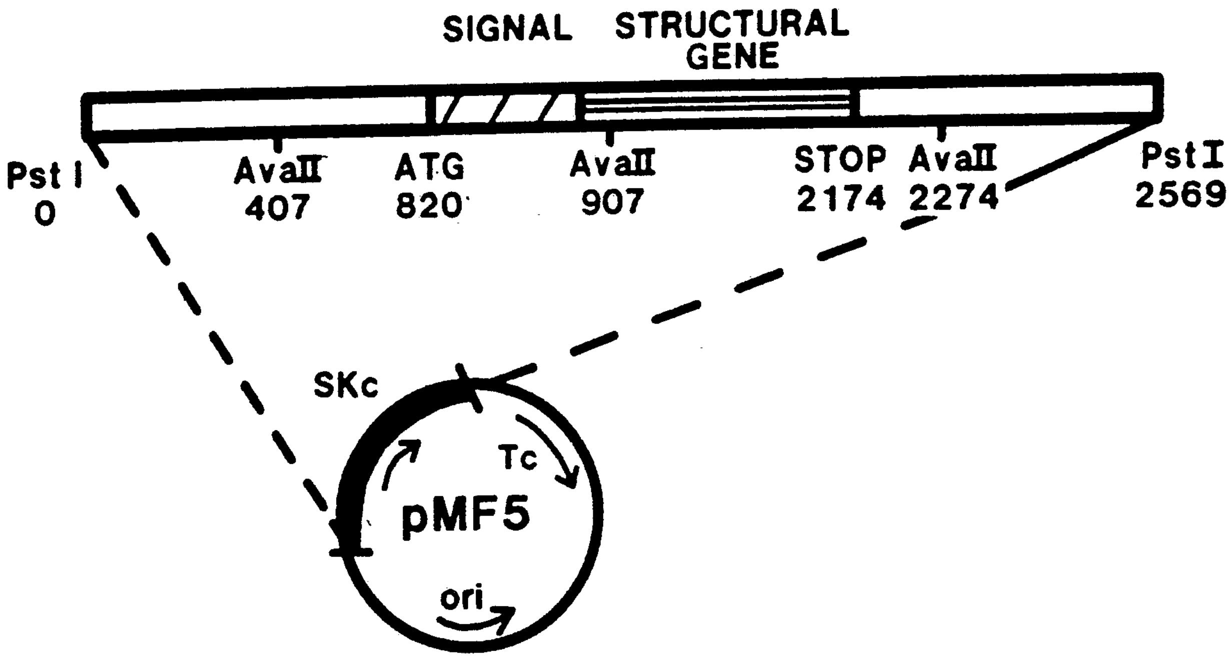
SIGNAL
STRUCTURAL GENE
Pst I 0
AvaII 407
ATG 820
AvaII 907
STOP 2174
AvaII 2274
PstI 2569
SKc
Tc
pMF5
ori

FIG. 6

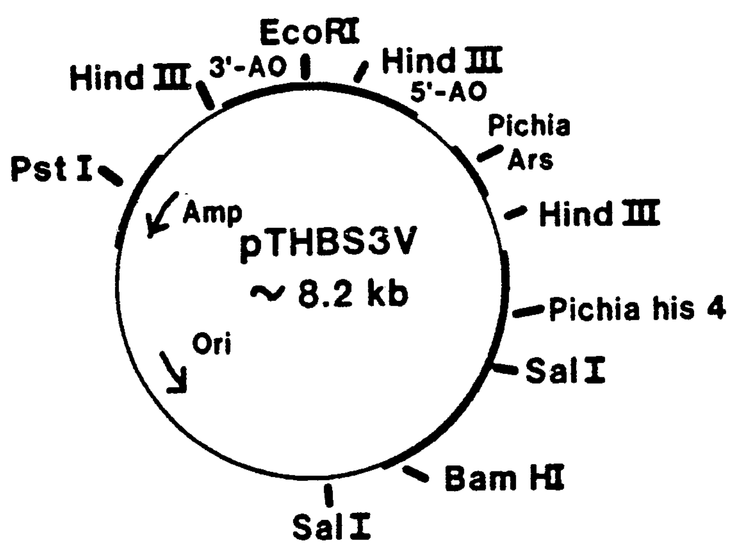
EcoRI
Hind III
3'-AO
Hind III
5'-AO
Pichia Ars
Pst I
Hind III
Amp
pTHBS3V
~ 8.2 kb
Pichia his 4
Ori
Sal I
Bam HI
Sal I

FIG. 7

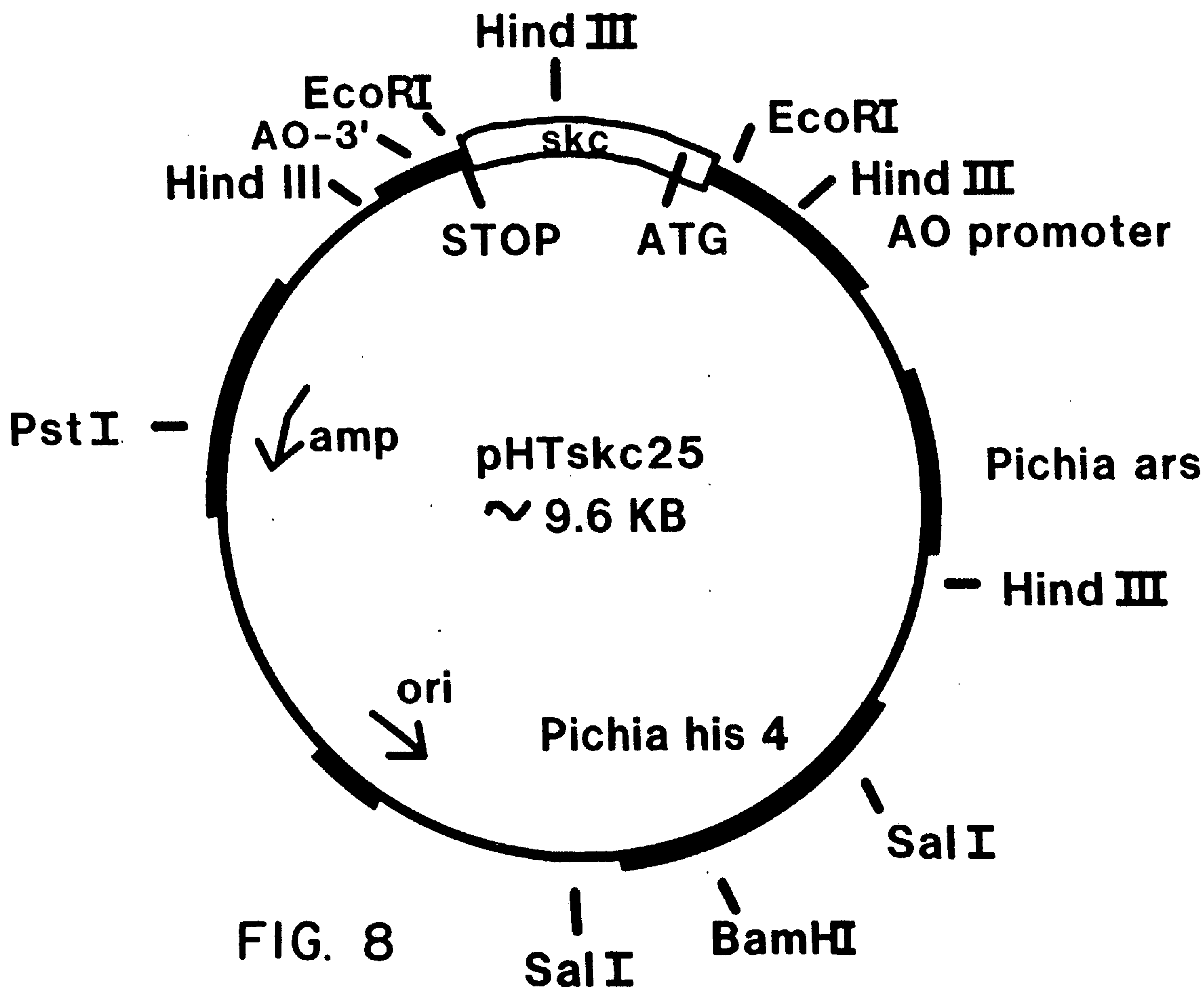
Hind III
EcoRI
AO-3'
Hind III
skc
EcoRI
Hind III
AO promoter
STOP
ATG
Pst I
amp
pHTskc25
~9.6 KB
Pichia ars
Hind III
ori
Pichia his 4
Sal I
BamHI
Sal I

FIG. 8

European Patent Office

# EUROPEAN SEARCH REPORT

Application number

EP 87 10 6614

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 151 337 (PHILLIPS PETROLEUM CO.)<br>* Page 3, lines 12-37; figure 2; claims *<br>--- | 1 | C 12 N 15/00<br>C 12 N 9/70<br>C 12 P 21/02<br>A 61 K 37/54 |
| Y | EP-A-0 150 424 (VEB ARZNEIMITTELWERK DRESDEN)<br>* Page 2, line 27 - page 3, line 29; claims *<br>--- | 1 | |
| Y | MOLECULAR AND CELLULAR BIOLOGY, vol. 5, no. 5, May 1985, pages 1111-1121, American Society for Microbiology; S.B. ELLIS et al.: "Isolation of alcohol oxidase and two other methanol regulatable genes from the yeast Pichia pastoris"<br>* Abstract; page 1118, column 1, line 6 - column 2, line 41; page 1119, column 1, lines 46-63; column 2, lines 34-49; figure 3 *<br>--- | 1,2 | TECHNICAL FIELDS SEARCHED (Int. Cl.4)<br>C 12 N |
| Y | MOLECULAR AND CELLULAR BIOLOGY, vol. 5, no. 12, December 1985, pages 3376-3385, American Society for Microbiology; J.M. CREGG et al.: "Pichia pastoris as a host system for transformations"<br>* Abstract; page 3376, column 1, line 1 - column 2, line 6; discussion *<br>--- -/- | 1,2 | |

The present search report has been drawn up for all claims

| Place of search | Date of completion of the search | Examiner |
|---|---|---|
| THE HAGUE | 24-08-1987 | YEATS S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 87 10 6614

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| Y | EP-A-0 143 081 (CIBA-GEIGY AG) * Abstract; page 6, lines 1-28; page 8, lines 4-30; claims * --- | 1,2 | |
| Y | EP-A-0 123 544 (GENENTECH, INC.) * Abstract; page 2, lines 19-36; example N; figure 10, claims * ----- | 1,2 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 24-08-1987 | YEATS S.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82